# EUROPEAN PATENT APPLICATION

(11) **EP 2 803 672 A1**
(43) Date of publication of application: **19.11.2014**
(21) Application number: 13736389.1
(22) Date of filing: 07.01.2013
(51) Int. Cl.: C07J 9/00, C08G 73/10, G02F 1/1337

(54) **LIQUID CRYSTAL ALIGNING AGENT**

(30) Priority: 12.01.2012 JP 2012003886
(71) Applicant: Wako Pure Chemical Industries, Ltd., Osaka-shi Osaka 540-8605 (JP)
(72) Inventor: KOZAKI Atsushi, Kawagoe-shi Saitama 350-1101 (JP); SATO Michihiko, Kawagoe-shi Saitama 350-1101 (JP)
(74) Representative: Winter, Brandl, Fürniss, Hübner, Röss, Kaiser, Polte - Partnerschaft mbB
(86) International application number: PCT/JP2013/050014
(87) International publication number: WO 2013/105523

(57) **Abstract**

A problem of the present invention is to provide a liquid crystal aligning agent which never deteriorates even in a method where UV is irradiated such as a liquid crystal dropping method or the like, and is capable of forming a liquid crystal alignment film having high UV resistance.

The present invention relates to,
(I) Compound represented by the following general formula [1] 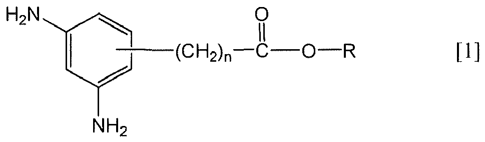 (wherein n represents an integer of 1 to 6; and R represents an alkyl group having 8 to 20 carbon atoms or a group having a steroid skeleton),
(II) Polyamic acid or a polyimide obtained by a reaction of
(A) compound represented by the above general formula [1], and the following compounds (B) and (C),
(B) compound represented by the following general formula [2], 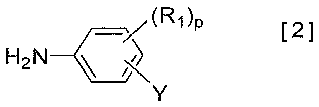 (wherein R₁ represents an alkyl group having 1 to 6 carbon atoms, or an arylalkyl group having 7 to 12 carbon atoms, p represents an integer of 0 to 4, and Y represents an amino group or a 4-aminophenyl group),
(C) compound represented by the following general formula [3] or [4], 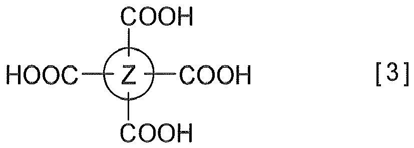 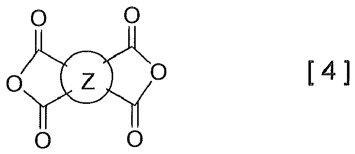 (wherein Z represents a tetravalent hydrocarbon group), along with
(III) Liquid crystal aligning agent comprising the polyamic acid or the polyimide obtained by a reaction of the above compounds (A), (B) and (C).

## Description

### TECHNICAL FIELD

The present invention relates to a liquid crystal aligning agent which makes it possible to produce a liquid crystal alignment film having high voltage retention rate even after UV irradiation, a polyamic acid or a polyimide which is a main component of said liquid crystal aligning agent, along with a compound to be used for producing said polyamic acid or polyimide.

### BACKGROUND ART

A cell step in a manufacturing process of a liquid crystal display has conventionally been performed by a method, wherein a liquid crystal is injected to the inside from an opening part, after making a cell, accommodating a liquid crystal substrate, vacuum, by utilization of its vacuum, that is, a vacuum injection method. Said method, however, had a problem of requiring the longer time in filling the liquid crystal to the inside of the cell, when size of a liquid crystal display becomes the larger. Accordingly, in recent years where demand of a liquid crystal display having a larger size has been increasing, instead of the vacuum injection method, a liquid crystal dropping method has been used where a liquid crystal display is manufactured by direct dropping of liquid crystal into each cell.

In said liquid crystal dropping method, firstly, after coating an alignment film onto a glass substrate, a UV sealing agent is dropped onto the substrate, and UV is irradiated onto the whole surface of the substrate to temporarily attach the UV sealing agent. After that, liquid crystal is dropped onto the substrate and the substrates is laminated and cured under heating, and a liquid crystal panel is manufactured. However, because UV is also irradiated onto the alignment film in the step for the above temporarily tacking the UV sealing agent, there has been a problem of deterioration of the alignment film by the UV irradiation and decrease in electrical characteristics such as voltage retention rate or the like.

### SUMMARY OF INVENTION

### PROBLEM TO BE SOLVED BY THE INVENTION

The present invention was completed in view of the above situation, and is to provide a liquid crystal aligning agent which does not deteriorate even in a method where UV is irradiated such as a liquid crystal dropping method, and makes it possible to form a liquid crystal alignment film having high UV resistance.

### MEANS FOR SOLVING THE PROBLEM

As a result of intensive study, the present inventors have found that by preparing a liquid crystal alignment film with using, as a raw material of a liquid crystal aligning agent, a compound represented by the following general formula [1]: 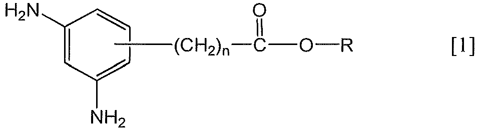 (wherein n represents an integer of 1 to 6, and R represents an alkyl group having 8 to 20 carbon atoms or a group having a steroid skeleton), a liquid crystal alignment film having high voltage retention rate even after UV irradiation, that is, a liquid crystal alignment film having high UV resistance can be attained, and have thus completed the present invention.

That is, the present invention relates to (I) Compound represented by the following general formula [1] 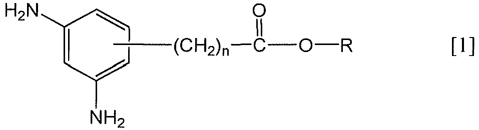 (wherein n represents an integer of 1 to 6; and R represents an alkyl group having 8 to 20 carbon atoms or a group having a steroid skeleton), (II) Polyamic acid or a polyimide obtained by a reaction of (A) compound represented by the above general formula [1], and the following compounds (B) and (C): (B) compound represented by the following general formula [2], 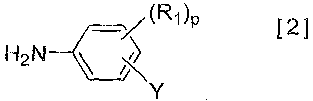 (wherein R₁ represents an alkyl group having 1 to 6 carbon atoms, or an arylalkyl group having 7 to 12 carbon atoms, p represents an integer of 0 to 4, and Y represents an amino group or a 4-aminophenyl group), (C) compound represented by the following general formula [3] or [4], 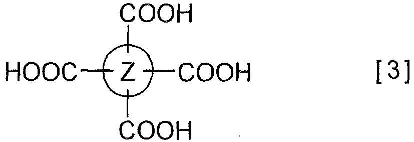 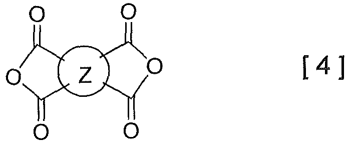 (wherein Z represents a tetravalent hydrocarbon group), along with (III) Liquid crystal aligning agent comprising the polyamic acid or the polyimide obtained by a reaction of the above compounds (A), (B) and (C).

### EFFECT OF THE INVENTION

The liquid crystal aligning agent of the present invention is the one containing a polyamic acid or a polyimide prepared by using the compound represented by the general formula [1] of the present invention, and the liquid crystal alignment film produced by said liquid crystal aligning agent has high voltage retention rate, as well as can suppress deterioration thereof even under strong UV irradiation. Therefore it has high voltage retention rate even after UV irradiation. Accordingly, the liquid crystal aligning agent of the present invention can use suitably in the liquid crystal dropping method where UV is irradiated onto the liquid crystal alignment film. Further, the liquid crystal alignment film produced by the liquid crystal aligning agent of the present invention is the one having a pre-tilt angle of 85° to 90 °in the liquid crystal display element, and is the one suitable for the vertical alignment liquid crystal display element.

### DESCRIPTION OF EMBODIMENTS

### (A) Compound represented by the general formula [1]

In the general formula [1], "n" represents an integer of 1 to 6, preferably an integer of 1 to 3, and more preferably an integer of 1 to 2.

The alkyl group having 8 to 20 carbon atoms, represented by R in the general formula [1], may be any of a straight, branched, or cyclic alkyl group, and includes, specifically, for example, an n-octyl group, an isooctyl group, a sec-octyl group, a tert-octyl group, a neooctyl group, a 2-ethylhexyl group, a cyclooctyl group, a n-nonyl group, an isononyl group, a sec-nonyl group, a tert-nonyl group, a neononyl group, a cyclononyl group, an n-decyl group, an isodecyl group, a sec-decyl group, a tert-decyl group, a neodecyl group, a cyclodecyl group, an n-undecyl group, a cycloundecyl group, an n-dodecyl group, a cyclododecyl group, an n-tridecyl group, a cyclotridecyl group, an n-tetradecyl group, a cyclotetradecyl group, an n-pentadecyl group, a cyclopentadecyl group, an n-hexadecyl group, a cyclohexadecyl group, an n-heptadecyl group, a cycloheptadecyl group, an n-octadecyl group, a cyclooctadecyl group, an n-nonadecyl group, a cyclononadecyl group, an n-icosyl group, a cycloicosyl group, a bornyl group (bornan-χ-yl group), an adamantyl group, a methyladamantyl group, a menthyl group (menthan-χ-yl group), a decahydronaphthyl group or the like. Among these alkyl groups, a straight alkyl group having 8 to 20 carbon atoms is preferable, and specifically, there is included an n-dodecyl group, an n-tridecyl group, an n-tetradecyl group, an n-pentadecyl group, an n-hexadecyl group, an n-heptadecyl group, an n-octadecyl group, an n-nonadecyl group, an n-icosyl group, as preferable alkyl groups.

As a group having the steroid skeleton represented by R in the general formula [1], there is included a group having a cyclopentahydrophenanthrene skeleton as a fundamental skeleton in which group at position 3 is a bond for binding with an oxygen atom in the general formula [1]. As a specific example thereof, there is included for example, those represented by the following [1'-1] to [1'-6] and the like (* in the above group represents a bond for binding with an oxygen atom in the general formula [1]). Among these groups having the steroid skeleton, a cholestaryl group ([1'-4]) is preferable.

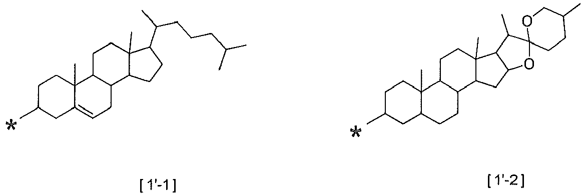

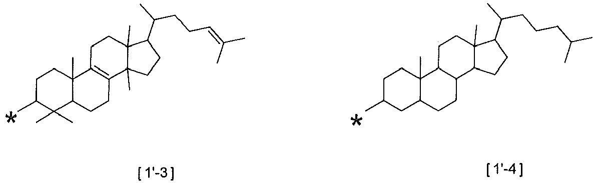

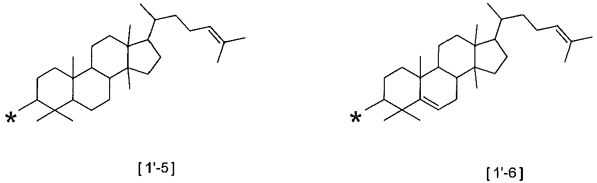

In the compound represented by the general formula [1], as for position of -(CH₂)ₙ-C=O-O-R binding to a diaminophenyl group, in the case that binding position of said group to a benzene ring is position 1, it is preferable that two amino groups are bonded in position 2 and position 4, or position 3 and position 5, and the bonding in the position 2 and the position 4 is more preferable.

As a specific example of the compound represented by the general formula [1], those represented by the following [1-1] to [1-44] are included, and among these, those represented by [1-1] to [1-12] are preferable, those represented by [1-1] to [1-12] are more preferable, and those represented by [1-1] to [1-3] are particularly preferable.

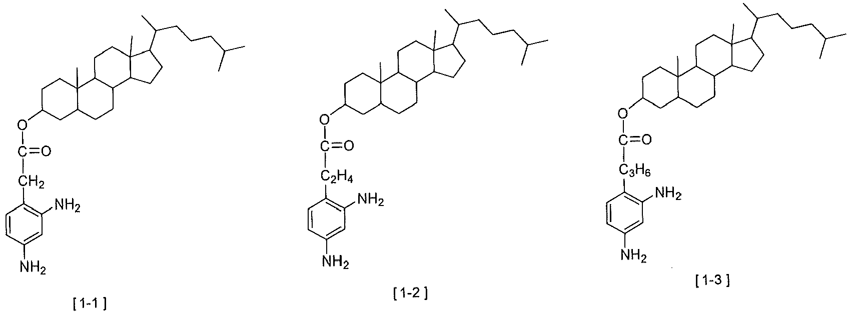

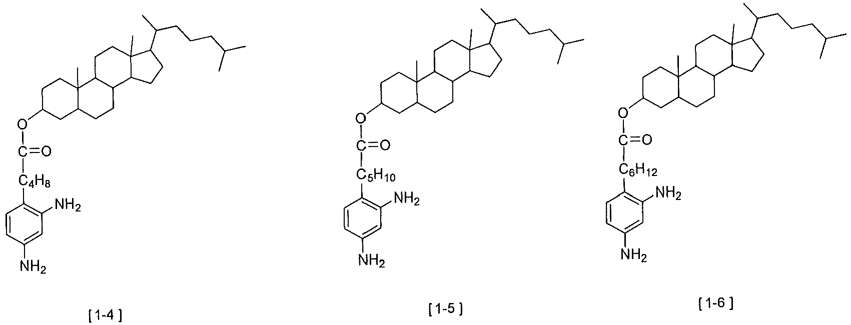

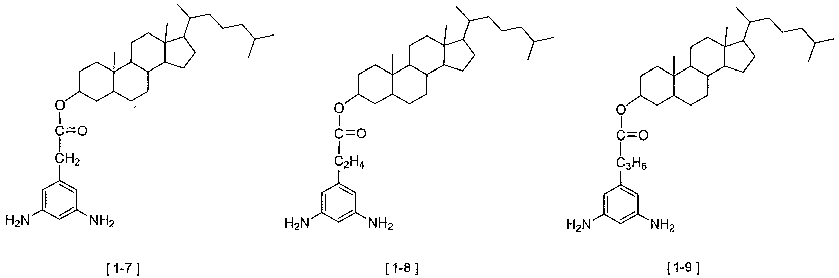

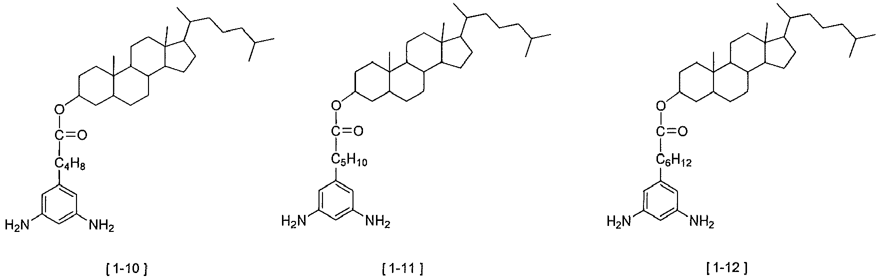

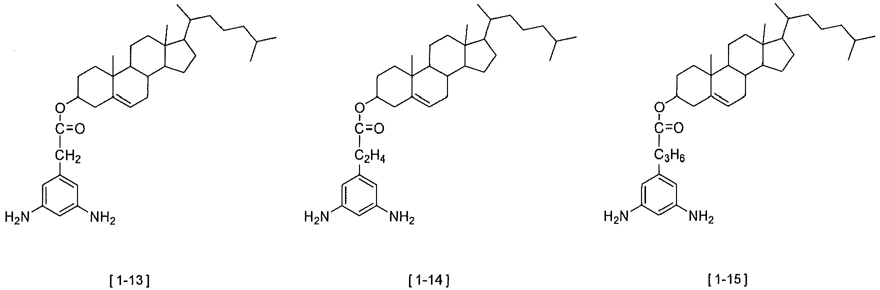

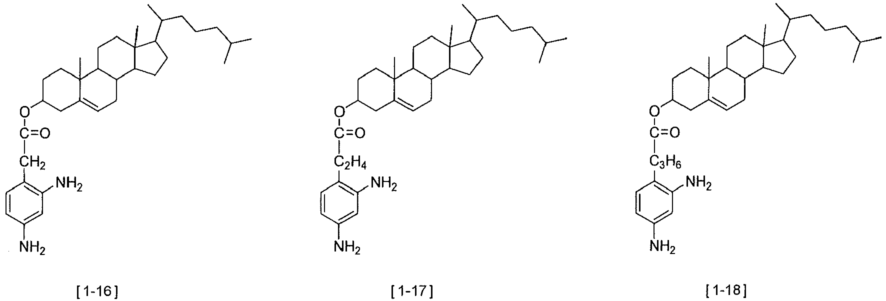

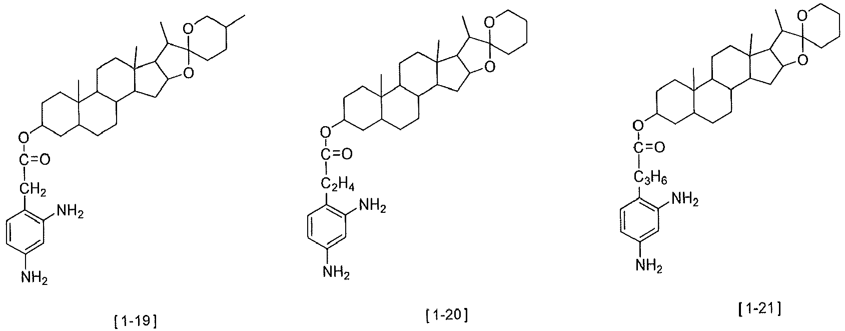

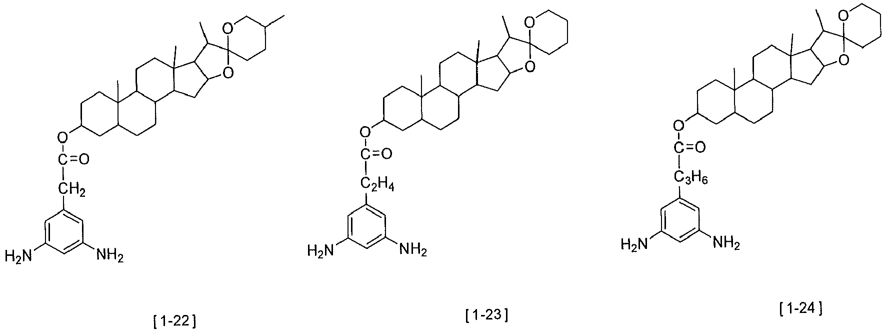

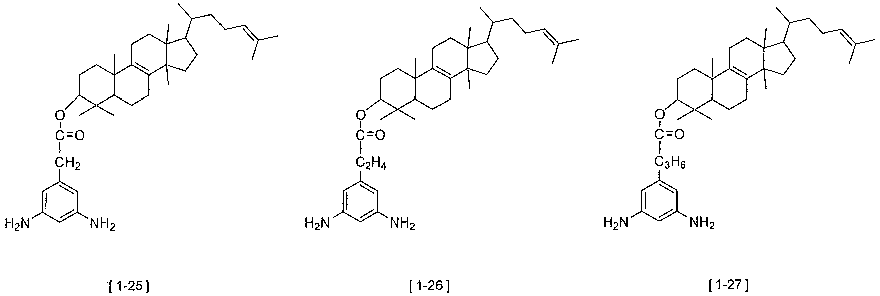

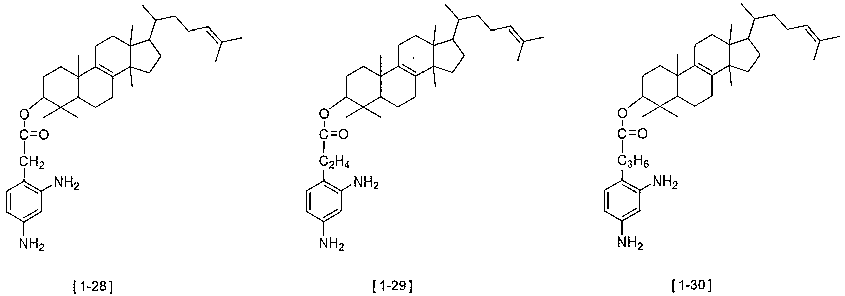

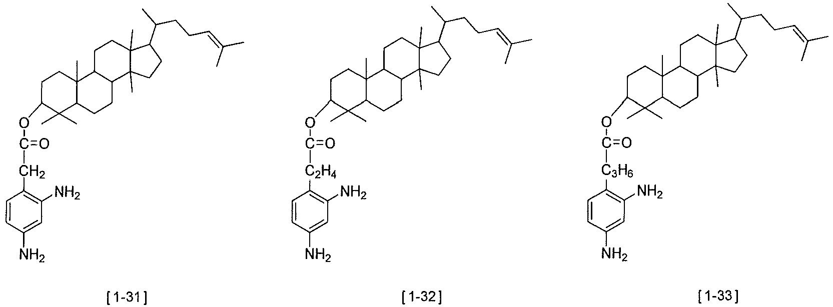

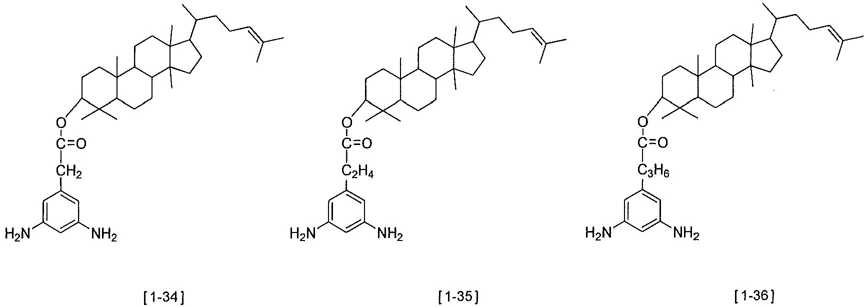

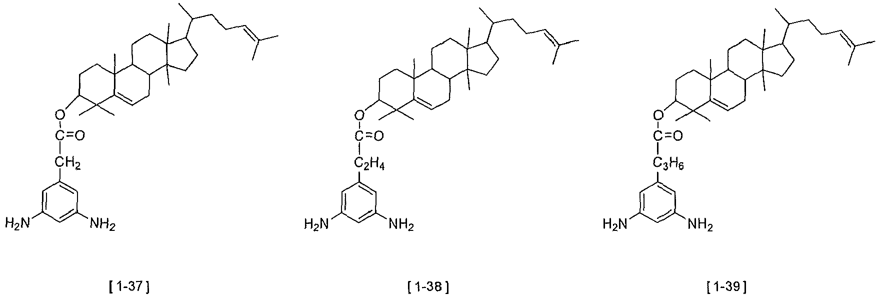

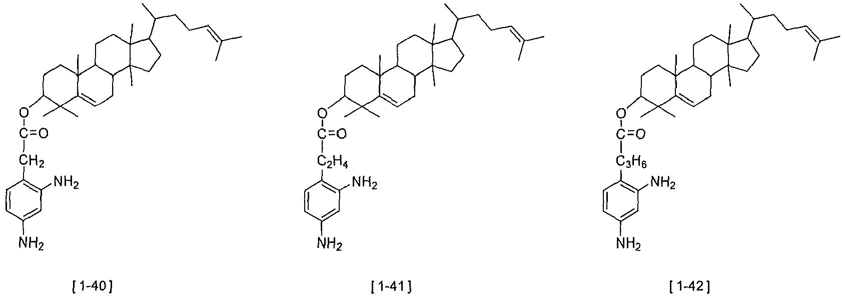

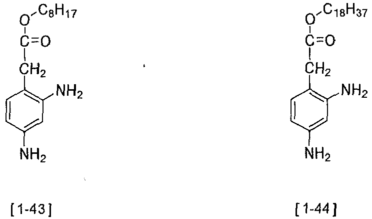

The compound represented by the above general formula [1] may be synthesized as appropriate, by a usual method, and may be synthesized, for example, in accordance with a method described in the first edition of New Experimental Chemistry Lecture (volume 14, page 1000 to 1062, page 1261 to 1300, page 1333 to 1341 and the like). Specifically, it is synthesized, for example, as follows.

That is, in the case where the R in the general formula [1] is a cholestaryl group, the synthesis is performed by a dehydration reaction of cholestanol and the compound represented by the following general formula [1']: 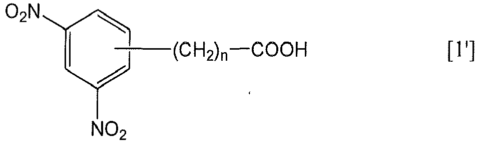 (wherein n is the same as above) in an appropriate solvent, under refluxing, if needed, and then by reducing a nitro group to an amino group by a reduction reaction. An organic solvent to be used in a dehydration reaction is not especially limited, and the one boiling azeotropically with water is preferable, including, for example, toluene, cyclohexane, hexane or the like. Reaction temperature of the dehydration reaction may be set as appropriate in response to a solvent to be used, and it is usually 20 to 150°C, and reaction time is usually 1 to 10 hours. The above reduction reaction is performed by adding a catalyst such as palladium, osmium, ruthenium which is usually used in this field, in an amount of usually 1.0 × 10⁻⁶ to 1.0 mol relative to 1 mol of the compound represented by the general formula [1'], at usually 20 to 80°C for usually 1 to 50 hours. An organic solvent in the reduction reaction is not especially limited, as long as it is the one usually used in this field, including, for example, alcohols such as methanol, ethanol, n-propanol, isopropanol, n-butanol, isobutanol, sec-butanol, tert-butanol; halogenated hydrocarbons such as dichloromethane, 1,2-dichloroethane, chloroform, carbon tetrachloride; ethers such as diethyl ether, dimethoxyethane, diethoxyethahe, tetrahydrofuran; hydrocarbon such as n-hexane, n-heptane, cyclohexane; aromatic hydrocarbons such as benzene, toluene, xylene; esters such as ethyl acetate, butyl acetate.

As the compound represented by the above general formula [1'], a commercially available product may be used, or it may also be synthesized in accordance with a known method. As the synthesis example thereof, for example, a phenylalkyl carboxylic acid having 8 to 13 carbon atoms may be reacted in an organic solvent such as chloroform with a mixed acid of sulfuric acid and nitric acid for 1 to 3 hours at 0 to 10°C, and it is obtained by purification as appropriate after the reaction.

In addition, in the case where the R in the general formula [1] is a cholestaryl group, as an another synthesis example of the compound represented by the general formula [1], it is achieved, for example, by an etherification reaction of cholestanol and the compound represented by the following general formula [1''] 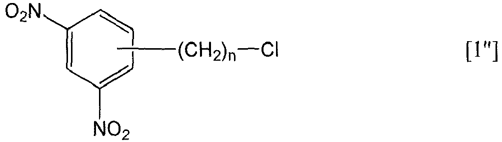 (wherein n is the same as above) in a suitable solvent, and then by reducing a nitro group to an amino group by a reduction reaction. An organic solvent to be used in an etherification reaction is not especially limited, and includes, for example, N,N-dimethylformamide, N-methylpyrrolidone, tetrahydrofuran, toluene or the like. Reaction temperature of the etherification reaction may be set as appropriate in response to a solvent to be used, and it is usually 20 to 150°C, and reaction time is usually 1 to 10 hours. In addition, as the above reduction reaction, the same method as the synthesis example, where the general formula [1'] was used, is included.

As the compound represented by the above general formula [1''], a commercially available product may be used, or it may also be synthesized in accordance with a known method. As the synthesis example thereof, it may be synthesized, for example, by a reaction of phenylalkyl chloride having 7 to 12 carbon atoms in the organic solvent such as chloroform, in a mixed acid of sulfuric acid and nitric acid for 1 to 3 hours at 0 to 10 °C, and by purification as appropriate after the reaction.

The compound represented by the above general formula [1] is used as a raw material of the polyamic acid or the polyimide for the liquid crystal aligning agent. The liquid crystal alignment film produced by the liquid crystal aligning agent, containing the polyamic acid or the polyimide obtained by using said compound, exerts effect that gives high voltage retention rate, as well as has little deterioration and can maintain high voltage retention rate, even after strong UV irradiation.

### (B) Compound represented by the general formula [2]

The alkyl group having 1 to 6 carbon atoms represented by R₁ in the general formula [2] may be any of a straight, branched, or cyclic alkyl group, and specifically, there is included for example, a methyl group, an ethyl group, an n-propyl group, an isopropyl group, an n-butyl group, an isobutyl group, a sec-butyl group, a tert-butyl group, a cyclobutyl group, an n-pentyl group, an isopentyl group, a sec-pentyl group, a tert-pentyl group, a neopentyl group, a 2-methylbutyl group, a 1,2-dimethylpropyl group, a 1-ethylpropyl group, a cyclopentyl group, an n-hexyl group, an isohexyl group, a sec-hexyl group, a tert-hexyl group, a neohexyl group, a 2-methylpentyl group, a 1,2-dimethylbutyl group, a 2,3-dimethylbutyl group, a 1-ethylbutyl group, a cyclohexyl group or the like. Among these alkyl groups, a methyl group, an ethyl group, an n-propyl group, and an isopropyl group are preferable, a methyl group is more preferable.

The arylalkyl group having 7 to 12 carbon atoms represented by R₁ in the general formula [1] includes a benzyl group, a phenylethyl group, a phenylpropyl group, a phenylbutyl group, a phenylpentyl group, a phenylhexyl group or the like, among them, a benzyl group is preferable.

"p" in the general formula [2] represents an integer of 0 to 4, and 0 or 1 is preferable, and 0 is preferable. In the case where p is an integer of 2 to 4, plural of R₁ may be same or different.

"Y" in the general formula [2] is preferably an amino group or a 4-aminophenyl group, and an amino group is more preferable. In addition, as a position of Y, it is preferable that Y is located at a para-position relative to the amino group.

As the compound represented by the general formula [2], specifically, the following formulae [2-1] to [2-8] and the like are included, and among them, the formulae [2-1] and [2-2] and the like are preferable, and the formula [2-1] is particularly preferable.

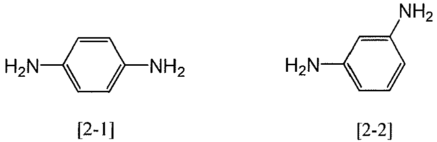

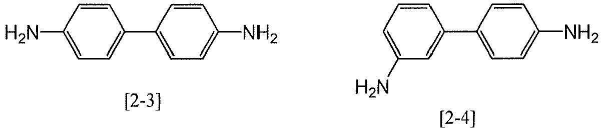

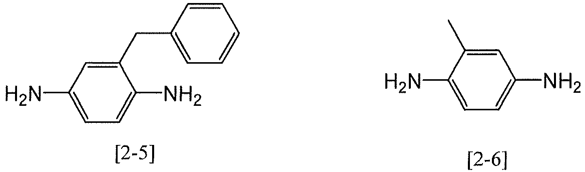

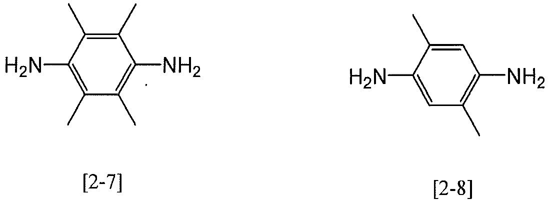

As the compound represented by the above general formula [2], a commercially available product may be used, or it may be synthesized by a known method per se, for example, in accordance with a method described in the first edition of New Experimental Chemistry Lecture (volume 14, pages 1261 to 1300, pages 1333 to 1341 and the like).

### (C) Compound represented by the general formula [3] or the general formula [4]

As the tetra-valent hydrocarbon group represented by Z in the compound represented by the general formula [3] or the general formula [4], for example, those represented by the following formulae [4-A] to [4-Z] and [4-a] to [4-i] are included, and among these, those represented by the formulae [4-G], [4-H], [4-J], [4-K] and the like are preferable, and the formula [4-H] is more preferable. 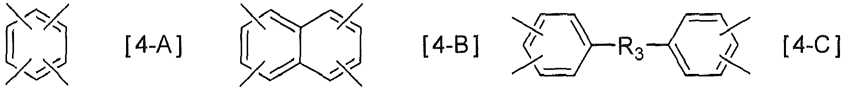 (wherein R₃ represents a bond, an oxygen atom, a methylene group, a perfluorodimethylmethylene group, a carbonyl group, or a sulfonyl group). 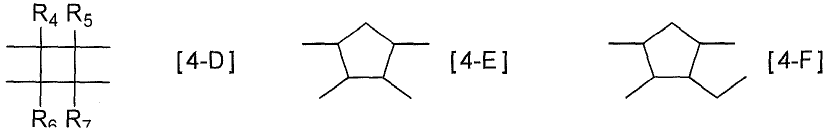 (wherein R₄ to R₇ represent each independently a hydrogen atom or an alkyl group having 1 to 3 carbon atoms). 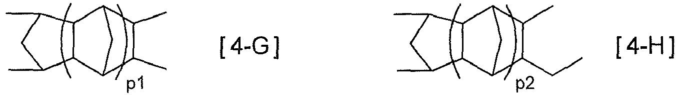 (wherein p1 and p2 represent 0 or 1) 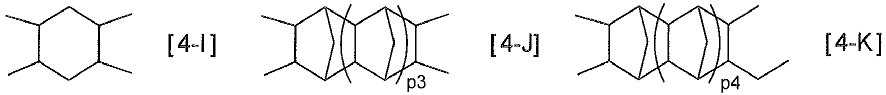 (wherein p3 and p4 represent 0 or 1) 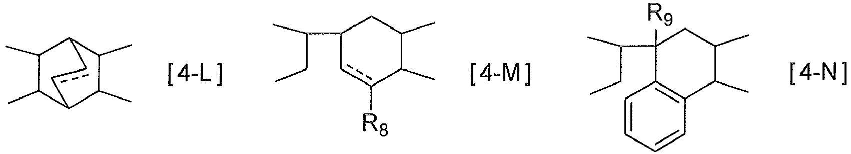 (wherein R₈ to R₉ represent each independently a hydrogen atom or an alkyl group having 1 to 3 carbon atoms, and a bonding represented by a solid line and a dotted line in the formulae means that it may be any of a single bond or a double bond). 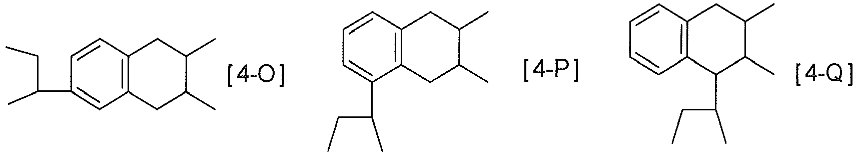 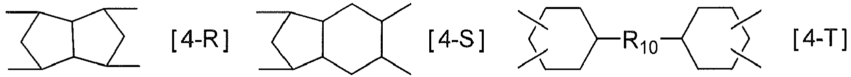 (wherein R₁₀ represents a bond, an oxygen atom, a sulfur atom, a methylene group or a sulfonyl group). 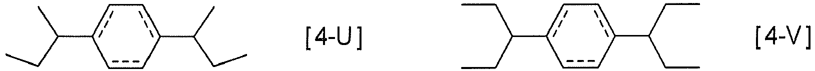 (wherein a bonding represented by a solid line and a dotted line in the formulae means that it may be any of a single bond or a double bond). 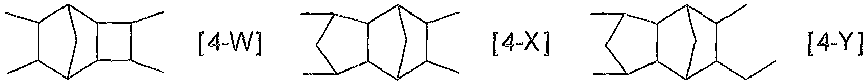 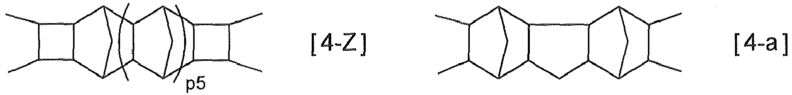 (wherein p5 represents 0 or 1) 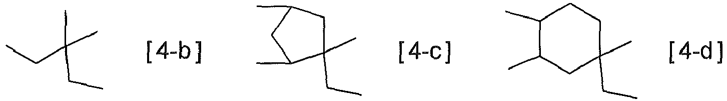 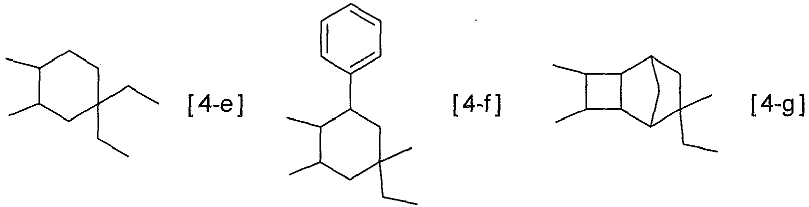 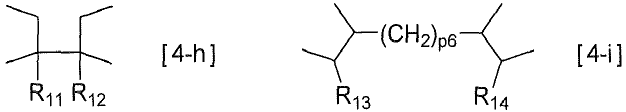 (wherein R₁₁ to R₁₄ represents each independently a hydrogen atom or an alkyl group having 1 to 3 carbon atoms, and p6 represents an integer of 8 to 20).

A bond represented by R₃ in the formula [4-C] represents that it binds both adjacent carbons, and in the case where R₃ is the bond, the formula [4-C] is shown below.

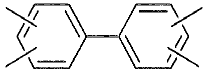

It should be noted that the bond in the present description means the similar one hereafter.

An alkyl group having 1 to 3 carbon atoms, represented by R₄ to R₇, R₈ to R₉, and R₁₁ to R₁₄, in the formulae [4-D], [4-M], [4-N], [4-h], and [4-i], may be any of a straight or branched alkyl group, and specifically, there is included, for example, a methyl group, an ethyl group, an n-propyl group, an isopropyl group. Among these alkyl groups, a methyl group is preferable.

R₄ to R₇, R₈ to R₉, and R₁₁ to R₁₄, in the formulae [4-D], [4-M], [4-N], [4-h], and [4-i] are preferably hydrogen atoms.

"p1" to "p5" in the formulae [4-G], [4-H], [4-J], [4-K], and [4-Z] include an integer of 0 or 1, and among them 0 is more preferable.

"p6" in the formula [4-i] includes an integer of 8 to 20.

The compound represented by the general formula [3] or the general formula [4] is more preferably the compound represented by the general formula [4].

A specific example of the compound represented by the general formula [3] or the general formula [4] includes the one derived from the above formulae [4-A] to [4-i], and specifically, includes those represented by the following formulae [3-1] to [3-61] and [4-1] to [4-61], and among these, those represented by the formulae [4-15], [4-17], [4-18], [4-19], [4-21] and the like are preferable, and the formula [4-18] is particularly preferable.

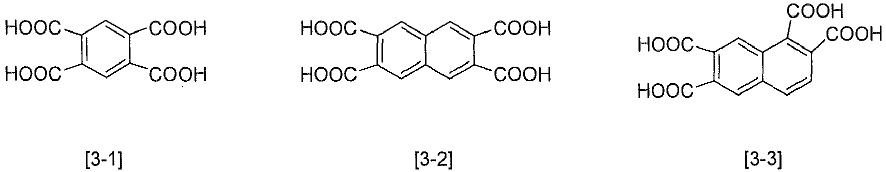

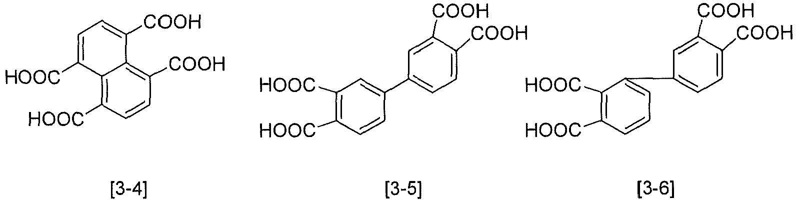

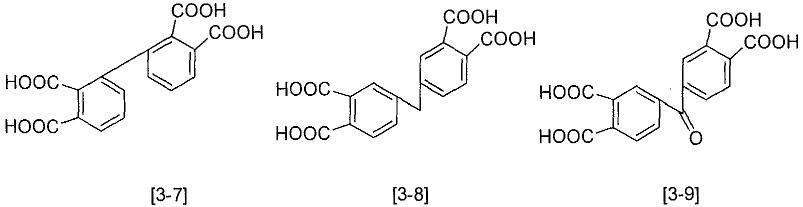

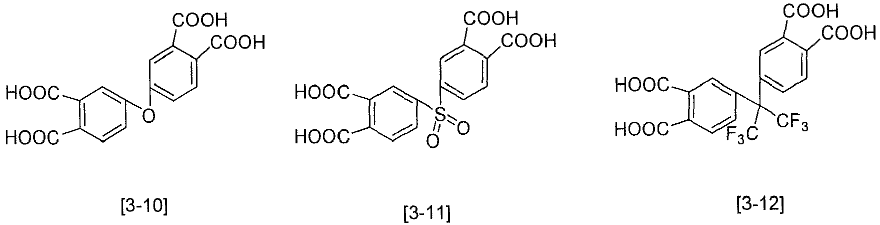

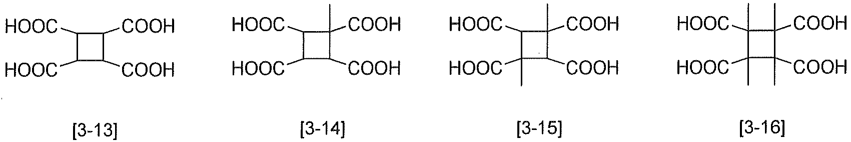

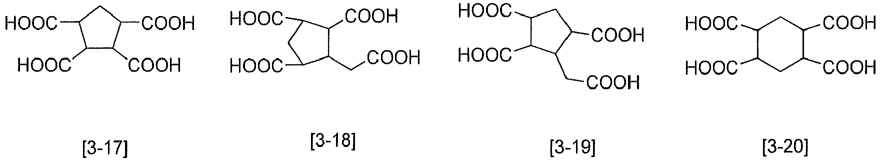

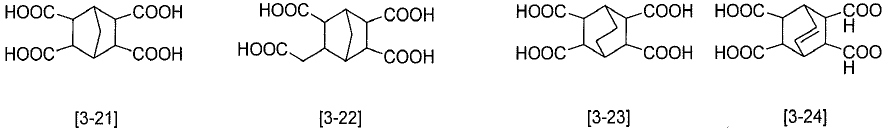

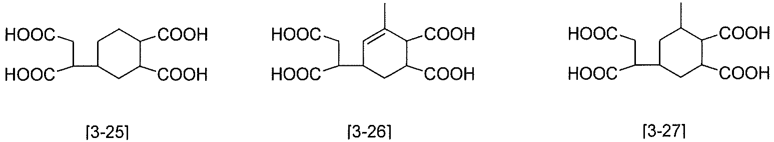

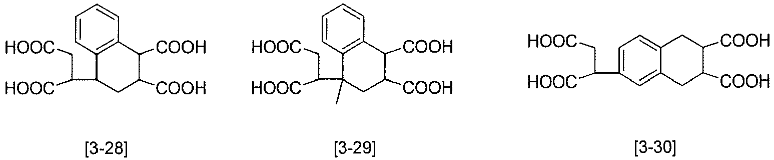

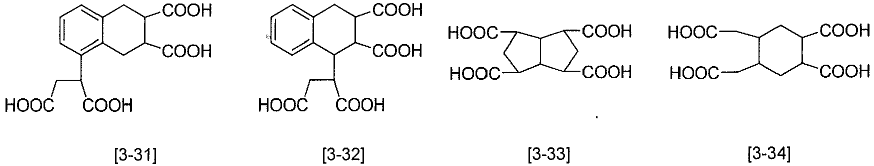

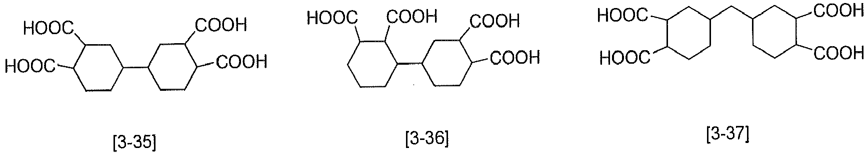

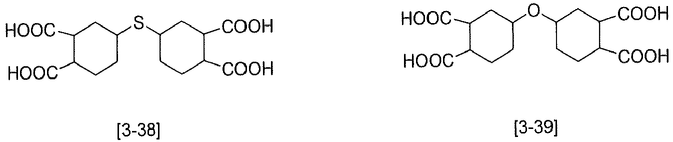

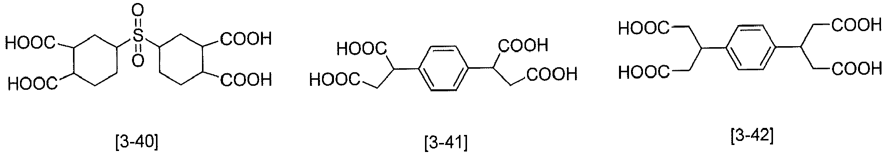

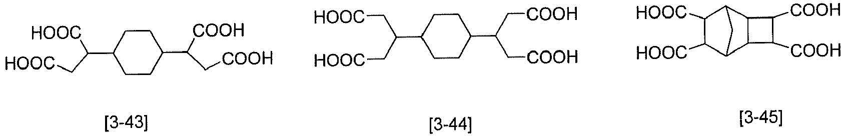

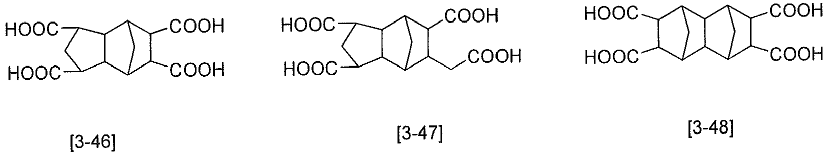

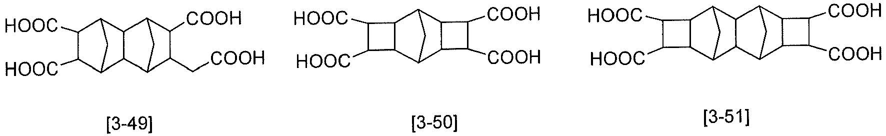

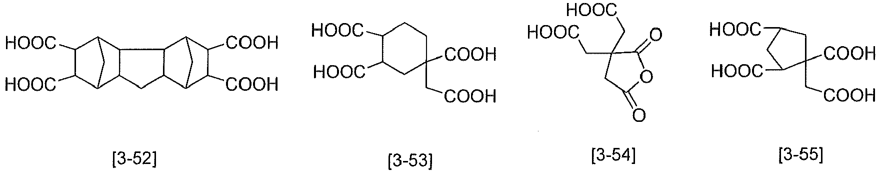

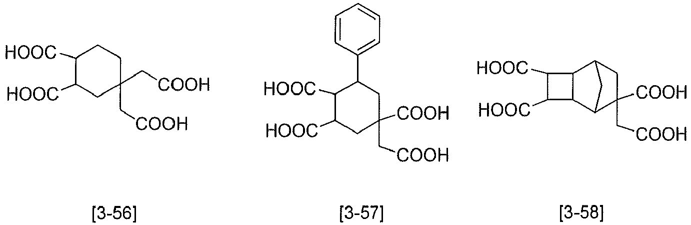

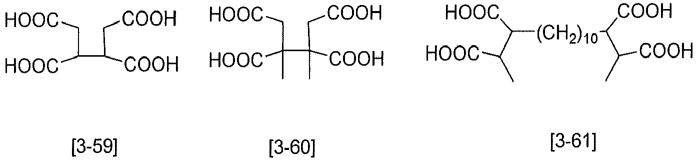

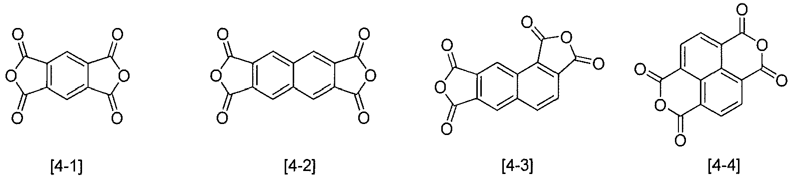

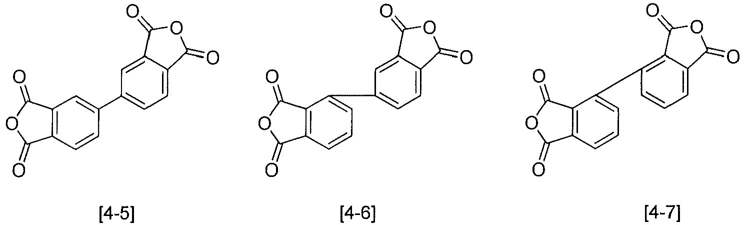

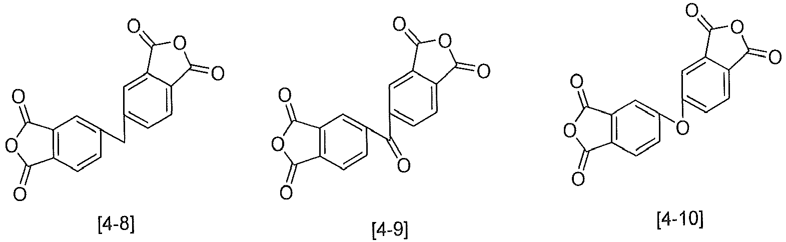

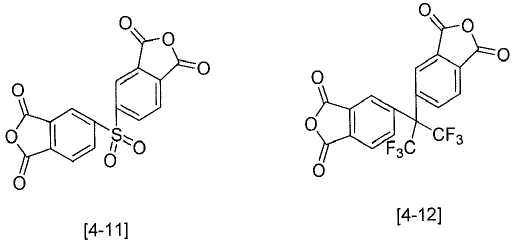

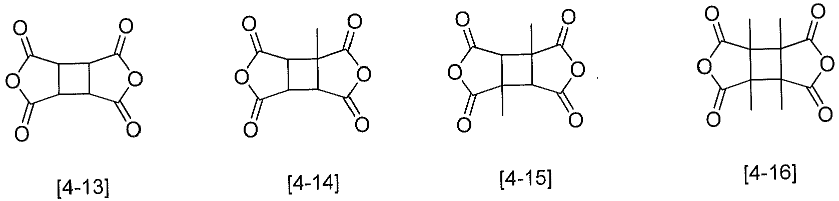

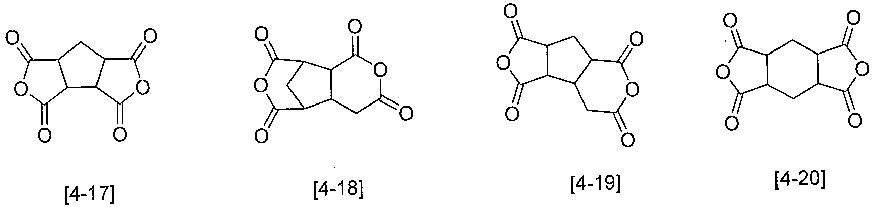

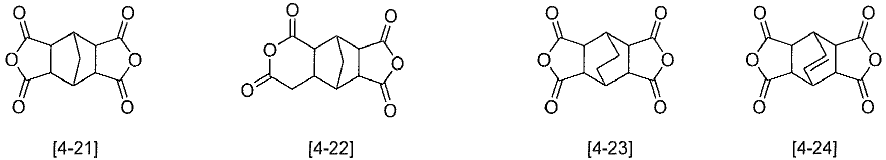

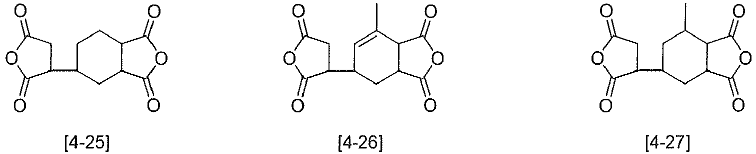

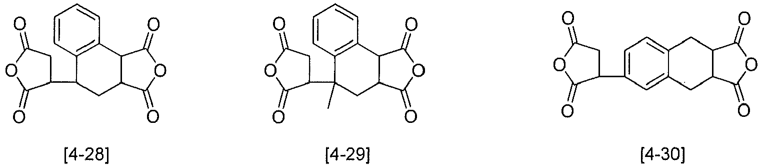

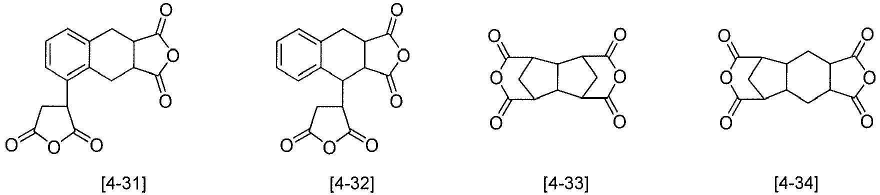

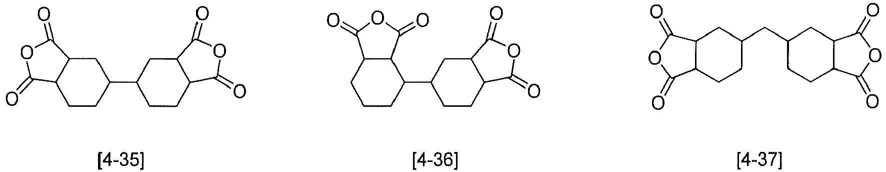

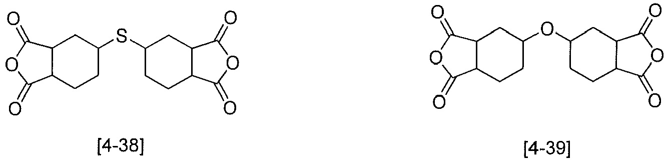

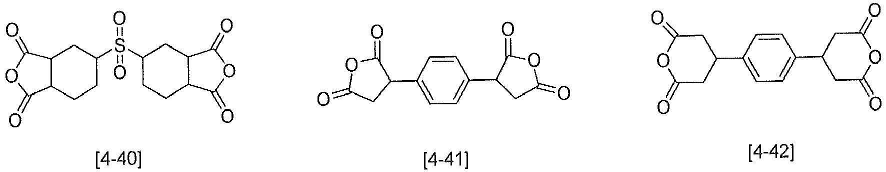

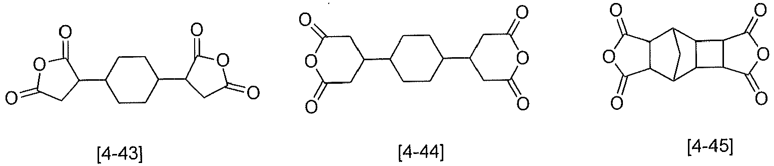

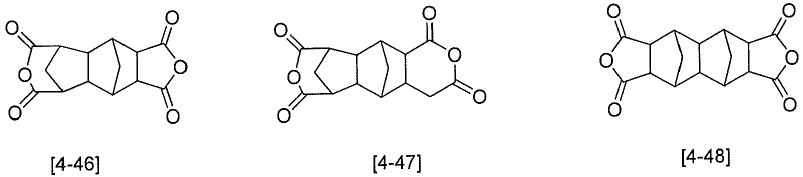

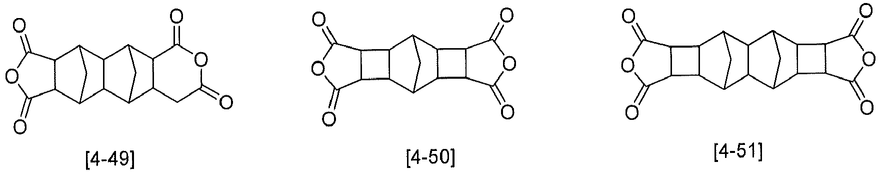

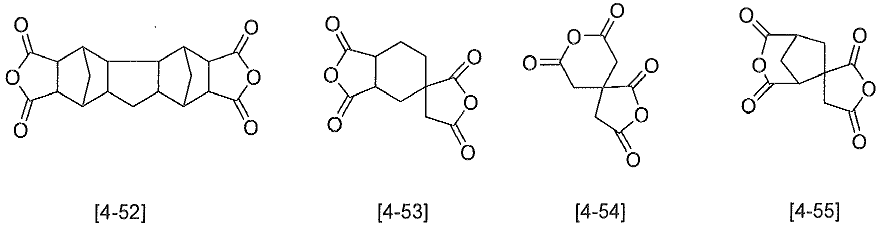

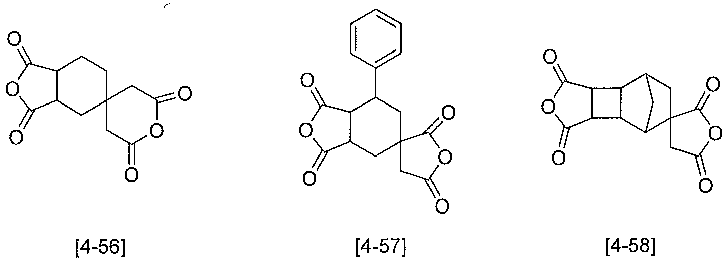

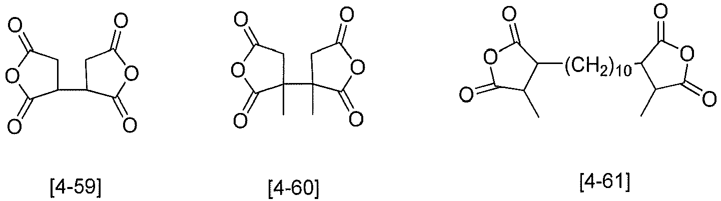

As the compound represented by the above general formula [3] or [4], a commercially available product may be used, or it may be synthesized by a known method per se, for example, by dehydration or the like of tetracarboxylic acid under heating, in accordance with a method described in the first edition of New Experimental Chemistry Lecture (volume 14, pages 1123 to 1133 and the like).

### Polyamic acid of the present invention

The polyamic acid of the present invention is the one obtained by a reaction of (A) the compound represented by the above general formula [1], (B) the compound represented by the general formula [2], along with (C) the compound represented by the general formula [3] or the general formula [4].

The polyamic acid of the present invention is synthesized specifically by a reaction of the compound represented by the general formula [1], the compound represented by the general formula [2], along with the compound represented by the general formula [3] or the general formula [4], in an organic solvent usually at 0 to 150°C and more preferably at 40 to 100°C, usually for 0.1 to 24 hours and preferably for 0.5 to 5 hours. Here, the organic solvent is not especially limitedas long as it is capable of dissolving a polyamic acid to be synthesized, and includes, for example, an aprotic polar solvent such as N-methyl-2-pyrrolidone, N,N-dimethylacetamide, N,N-dimethylformamide, N,N-dimethyl- imidazolidinone, dimethylsulfoxide, γ-butyrolactone, tetramethylurea, hexamethylphosphortriamideor the like; a phenolic solvent such as m-cresol, xylenol, phenol, halogenated phenol or the like, and includes N-methyl-2-pyrrolidone preferably. These organic solvents may be used alone or in combination of two or more kinds. The organic solvent may be used in such an amount that sum total amount of the compound represented by the above general formula [1], the compound represented by the general formula [2], along with the compound represented by the general formula [3] or the general formula [4] becomes usually 0.1 to 50% by weight, and preferably 10 to 50% by weight, relative to total amount of the reaction solution. It should be noted that the use amount of the organic solvent may be set in response to polymerization activity of the compound represented by the general formula [1], and for example, in the case where polymerization activity of the compound represented by the general formula [1] is high, it may be set so that the above sum total amount becomes low, while in the case where polymerization activity is low, it may be set so that the above sum total amount becomes high.

Ratio of use amount of the compound represented by the general formula [1] and the compound represented by the general formula [2] is usually 5:95 to 40:60, and preferably 10:90 to 30:70, in molar ratio. In addition, ratio of use amount of the compound represented by the general formula [1] and the compound represented by the general formula [2], and use amount of the compound represented by the general formula [3] or the general formula [4] is usually 10:9 to 10:11, and preferably 1:1, in molar ratio.

As stated above, a reaction solution containing the polyamic acid is obtained. Said reaction solution may be supplied as it is to prepare the liquid crystal aligning agent, or may be supplied, after isolation of the polyamic acid contained in the reaction solution, to prepare the liquid crystal aligning agent, or may be supplied, after purification of the isolated polyamic acid, to prepare the liquid crystal aligning agent. The isolation of the polyamic acid is performed by pouring the reaction solution into a large quantity of a poor solvent of the polyamic acid to obtain a precipitate, and by removing the solvent from this precipitate under reduced pressure. The poor solvent to be used here includes, for example, methanol, ethanol, isopropanol, cyclohexanol, ethylene glycol, propylene glycol, 1,4-butanediol, triethylene glycol, ethylene glycol monomethyl ether, ethyl lactate, butyl lactate, acetone, methyl ethyl ketone, methyl isobutyl ketone, cyclohexanone, methyl acetate, ethyl acetate, butyl acetate, methyl methoxypropionate, ethyl ethoxypropionate, diethyl oxalate, diethyl malonate, diethyl ether, ethylene glycol methyl ether, ethylene glycol ethyl ether, ethylene glycol-n-propyl ether, ethylene glycol-i-propyl ether, ethylene glycol-n-butyl ether, ethylene glycol dimethyl ether, ethylene glycol ethyl ether acetate, diethylene glycol dimethyl ether, diethylene glycol diethyl ether, diethylene glycol monomethyl ether, diethylene glycol monoethyl ether, diethylene glycol monomethyl ether acetate, diethylene glycol monoethyl ether acetate, tetrahydrofuran, dichloromethane, 1,2-dichloroethane, 1,4-dichlorobutane, trichloroethane, chlorobenzene, o-dichlorobenzene, hexane, heptane, octane, benzene, toluene, xylene or the like.

### Polyimide of the present invention

The polyimide of the present invention is produced by imidation by dehydration ring closure of an amic acid structure of the above polyamic acid of the present invention. The polyimide of the present invention contains two kinds, that is, a complete polyimide where the polyamic acid is completely imidized, and a partial polyimide where the polyamic acid is partially imidized. It should be noted that in the case of using the polyamic acid of the present invention as an application of the liquid crystal alignment film, use of the partial polyimide is preferable.

The dehydration ring closure of the polyamic acid is performed by either a method for heating the polyamic acid, or a method for dissolving the polyamic acid into an organic acid, and adding a dehydration agent or a catalyst for dehydration ring closure into this solution, and heating if necessary. The latter method is preferable.

Reaction temperature in the method for heating the polyamic acid is usually 50 to 200°C, and more preferably 80 to 150°C. The reaction temperature below 50°C may not provide sufficient progress of the dehydration ring closing reaction, while the reaction temperature over 200°C may decrease molecular weight of a polyimide obtained. Reaction time in the method for heating the polyamic acid is preferably 0.5 to 48 hours, and more preferably 1 to 20 hours.

In a method for adding the dehydration agent or the catalyst for dehydration ring closure into the solution of the polyamic acid, the dehydrating agent includes, for example, an acid anhydride etc. such as acetic anhydride, propionic anhydride, trifluoroacetic anhydride. Use amount of the dehydration agent is usually 0.01 to 20 moles relative to 1 mole of the amic acid structure unit. In addition, the catalyst for dehydration ring closure includes, for example, a tertiary amine etc. such as triethyl amine, pyridine, collidine. Use amount of the catalyst for dehydration ring closure is preferably set at 0. 1 to 10 moles relative to 1 mole of the dehydration agent to be used. In addition, concentration of the polyamic acid in the solution of the polyamic acid is usually 10 to 40% by weight, and preferably 10 to 20% by weight. In addition, as said solution, the same organic solvent as used in synthesis of the polyamic acid is included. Reaction temperature of the dehydration ring closing reaction is usually 50 to 200°C, and preferably 80 to 150°C, and reaction time is usually 0.5 to 20 hours, and more preferably 1 to 8 hours.

Imidation ratio in the polyimide of the present invention is usually 30 to 100%, preferably 40 to 80%, and more preferably 60 to 70%. By preparing a partial polyimide having the imidation ratio of such a range, expression of effect of good printability can be expected. This imidation ratio can be controlled by adjusting the above reaction conditions (dehydration agent, catalyst amount, reaction temperature and reaction time).

The polyimide of the present invention is stored usually in an organic solvent, and as said organic solvent, the same organic solvent as used in synthesis of the above polyamic acid is included. In addition, solid content concentration in this case is usually 15 to 25% by weight.

Viscosity of the polyimide of the present invention is usually 5 to 50 mPa·s, and preferably 5 to 30 mPa·s. It should be noted that said viscosity can be adjusted by reaction time of the imidation reaction and concentration of the polyimide, and objective viscosity may be attained by this adjustment.

### Liquid crystal aligning agent of the present invention

The liquid crystal aligning agent of the present invention is the one containing at least one or more kinds of the above polyamic acid and the polyimide of the present invention. It may contain other components. As other components it may include, for example, a functional silane containing-compound, or an epoxy-based cross-linking agent or the like.

The above functional silane-containing compound includes, for example, 3-aminopropyltrimethoxysilane, 3-aminopropyltriethoxysilane, 2-aminopropyltrimethoxysilane, 2-aminopropyltriethoxysilane, N-(2-aminoethyl)-3-aminopropyltrimethoxysilane, N-(2-aminoethyl)-3-aminopropylmethyldimethoxysilane, 3-ureidopropyltrimethoxysilane, 3-ureidopropyltriethoxysilane, N-ethoxycarbonyl-3-aminopropyltrimethoxysilane, N-ethoxycarbonyl-3-aminopropyltriethoxysilane, N-triethoxysilylpropyltriethylenetriamine, N-trimethoxysilylpropyltriethylenetriamine, 10-trimethoxysilyl-1,4,7-triazadecane, 10-triethoxysilyl-1,4,7-triazadecane, 9-trimethoxysilyl-3,6-diazanonyl acetate, 9-triethoxysilyl-3,6-diazanonyl acetate, N-benzyl-3-aminopropyltrimethoxysilane, N-benzyl-3-aminopropyltriethoxysilane, N-phenyl-3-aminopropyltrimethoxysilane, N-phenyl-3-aminopropyltriethoxysilane, N-bis(oxyehthylene)-3-aminopropyltrimethoxysilane, N-bis(oxyehthylene)-3-aminopropyltriethoxysilane or the like.

The above epoxy-based cross-linking agent includes polyethylene glycol diglycidyl ether, diglycidyl ortho-toluidine, tetraglycidylaminodiphenylmethane, 1,3-bis(N,N'-diglycidylaminomethyl)cyclohexane or the like, and tetraglycidylaminodiphenylmethane or the like is preferable.

The above functional silane-containing compound and the epoxy-based cross-linking agent may be added so that content thereof becomes usually 5 to 20% by weight relative to the polyimide.

A solvent to be used in preparing the liquid crystal aligning agent of the present invention, as a solution state, is not especially limited, as long as it is an organic solvent which dissolves the above-described polyamic acid or the polyimide of the present invention, and other components to be contained arbitrarily, and does not react with these. As such a solvent, for example, the same organic solvent as used in synthesis of the above polyamic acid of the present invention is included. These organic solvents may be used alone or in combination of two or more kinds. In addition, in the case where the liquid crystal aligning agent is coated by a spinner method, in order to enhance coating properties, an organic solvent containing 30 to 60%, and preferably 40 to 50% of butylcellosolve (ethylene glycol monobutyl ether) is preferable.

Solid content concentration of the liquid crystal aligning agent of the present invention, that is, ratio of weight of total components other than the solvent in the liquid crystal aligning agent, occupying in total amount of the liquid crystal aligning agent, is setin consideration of viscosity, volatility and the like, and it is preferably 1 to 10% by weight. The liquid crystal aligning agent of the present invention is coated at the substrate surface to form a coating film to become the liquid crystal alignment film, and in the case where the solid content concentration is below 1% by weight, thickness of this coating film becomes too thin, which may provide difficulty in obtaining a good liquid crystal alignment film, in some cases. On the other hand, in the case where the solid content concentration is over 10% by weight, thickness of coating film becomes too thick, which may provide difficulty in obtaining a good liquid crystal alignment film, as well as viscosity of the liquid crystal aligning agent increases, which may provide insufficient coating characteristics, in some cases.

The liquid crystal alignment film produced by using the above liquid crystal aligning agent of the present invention is the one which exerts superior effect of providing high voltage retention rate, as well as maintaining high voltage retention rate even after strong UV irradiation.

Explanation will be given below specifically on the present invention, based on Examples, however, the present invention should not be limited to these Examples.

### EXAMPLES

### Example 1 Synthesis of cholestanyl 2,4-diaminophenylacetate (D1)

### (1) Synthesis of cholestanyl 2,4-dinitrophenylacetate

By dissolving 34.37 g (88.4 mmol) of cholestanol (produced by Nikko Chemicals Co., Ltd.), 21.00 g (92.6 mmol) of 2,4-dinitrophenylacetic acid (produced by Tokyo Chemical Industry Co. , Ltd.), and 1.68 g (8.84 mmol) of toluene sulfonic acid monohydrate (produced by Wako Pure Chemical Industries Co. , Ltd.) in 264 ml of toluene, and stirring for 3 hours under refluxing, it was then confirmed that cholestanol became 1% or less, using ¹H-NMR. Next, after cooling the reaction product down to room temperature, 400 ml of methanol was injected and it was subjected to crystallization for 1 hour at 3 to 5°C. Then the resultant precipitate was filtered out and dried to obtain 46.48 g (77.9 mmol, yield: 88%) of cholestanyl 2,4-dinitrophenylacetate. Measurement result of ¹H-NMR of cholestanyl 2,4-dinitrophenylacetate is shown below.
¹H-NMR (400MHz, CDCl₃) δ: 8.94 ppm (1H, d, J = 2.4 Hz), 8.43 ppm (1H, dd, J = 8.0 and 2.4 Hz), 7.59 ppm (1H, d, J = 8.0 Hz), 4.73 ppm (1H, m), 4.11 ppm (2H, s), 1.94-0.64 ppm (47H, m)

### (2) Synthesis of cholestanyl 2,4-diaminophenylacetate

After dissolving 30.00 g (50.3 mmol) of cholestanyl 2,4-dinitrophenylacetate obtained in the above (1) into 150 ml of tetrahydrofuran (THF), inside of container was replaced with nitrogen, and 3.00 g of 5% Pd/C (50% wet, produced by N. E. Chemcat Corp.) was charged. After that, the inside of the container was replaced with hydrogen and a reaction was performed under stirring at room temperature for 40 hours. After confirming disappearance of the raw material using thin layer chromatography, Pd/C was filtered off. Next, after performing vacuum concentration, purification was performed using silica gel column chromatography (eluting solvent: ethyl acetate/toluene=1/2). The residue (22 g) was dissolved into 100 ml of dichloromethane and washed five times with 100 ml of ion exchanged water. Further, after performing vacuum concentration of the organic layer, the residue (21 g) was dissolved in 126 ml of THF, and 252 ml of methanol were injected. After crystallization by injecting 14 ml of ion exchanged water, stirring was continued for 1 hour at room temperature and further at 3 to 5°C for 1 hour, and by filtering out and drying, 18.91 g (35.2 mmol) of cholestanyl 2,4-diaminophenylacetate (D1) was obtained (yield: 70%). Measurement result of ¹H-NMR of cholestanyl 2,4-diaminophenylacetate is shown below.
¹H-NMR (400MHz, CDCl₃) δ: 6.85 ppm (1H, d, J = 7.8 Hz), 6.10 ppm (1H, dd, J = 7.8 and 2.2 Hz), 6.06 ppm (1H, d, J = 2.2 Hz), 4.66 ppm (1H, m), 3.75 ppm (4H, br s), 3.40 ppm (2H, s), 1.97 - 062 ppm (47H, m)

### Example 2 Synthesis of cholestanyl 2,4-diaminophenylpropionic acid (D2)

### (1) Synthesis of 2,4-dinitrophenylpropionic acid

50.66 g (337 mmol) of phenylpropionic acid (produced by Wako Pure Chemical Industries Co., Ltd.) was dissolved in 245 ml of chloroform, and then an mixed acid of 661.75 g (6.75 mol) of concentrated sulfuric acid (produced by Wako Pure Chemical Industries Co., Ltd.) and 94.47 g (1.35 mol) of fuming nitric acid (produced by Wako Pure Chemical Industries Co., Ltd.) was dropped at a temperature of below 10°C, under cooling with ice. After stirring for 1 hour under ice cooling, disappearance of the raw material was confirmed using NMR. After completion of the reaction, the reaction solution was injected into 400 ml of ice water to separate the solution with 250 ml of ethyl acetate. After washing the organic layer four times with 250 ml of ion exchanged water, it was concentrated and the residue was washed under kneading with 160 ml of ion exchanged water. Next, it was filtered out and dried to obtain 74.76 g (311 mmol) of 2,4-dinitrophenylpropionic acid (yield: 92%). Measurement result of ¹H-NMR of 2,4-dinitrophenylpropionic acid is shown below.
¹H-NMR (400MHz, CDCl₃) δ: 8.82 ppm (1H, d, J = 2.4 Hz), 8.40 ppm (1H, dd, J = 8.5 and 2.4 Hz), 7.69 ppm (1H, d, J = 8.5 Hz), 3.34 ppm (2H, t, J = 7.3 Hz), 2.84 ppm (2H, t, J = 7.3 Hz)

### (2) Synthesis of cholestanyl 2,4-dinitrophenylpropionic acid

60.00 g (250 mmol) of 2,4-dinitrophenylpropionic acid obtained in above (1), 101.95 g (262mmol) of cholestanol, and 4.75 g (25.0 mmol) of toluene sulfonic acid monohydrate were suspended in 763 ml of toluene, and after stirring the suspension for 3 hours under refluxing, it was confirmed that cholestanol became 5% or less, using ¹H-NMR. After concentration, it was washed under kneading with 610 ml of methanol for 30 minutes and filtered out. The resultant filtrate was washed under kneading at 50°C for 1 hour with 660 ml of methanol, and further washed under kneading at room temperature for 1 hour, and filtered out. After that the crystal was further washed under kneading at 50°C for 1 hour and at room temperature for 1 hour with 660 ml of methanol to obtain 131.58 g (215 mmol) of cholestanyl 2,4-dinitrophenylpropionic acid (yield: 86%). Measurement result of ¹H-NMR of cholestanyl 2,4-dinitrophenylpropionic acid is shown below.
¹H-NMR (400MHz, CDCl₃) δ: 8.80 ppm (1H, d, J = 2.4 Hz), 8.37 ppm (1H, dd, J = 8.6 and 2.4 Hz), 7.68 ppm (1H, d, J = 8.6 Hz), 4.70 ppm (1H, m), 3.32 ppm (2H, t, J = 7.3 Hz), 2.73 ppm (2H, t, J = 7.3 Hz), 1.97-0.64 ppm (47H, m)

### (3) Synthesis of cholestanyl 2,4-diaminophenylpropionic acid

After dissolving 50.00 g (81.9 mmol) of 2,4-dinitrophenylpropionic acid obtained in the above (2) into 250 ml of THF, inside of container was replaced with nitrogen, and 5.00 g of 5% Pd/C was charged. After that, inside of container was replaced with hydrogen and a reaction was performed under stirring at room temperature for 21 hours. After confirming disappearance of the raw material using thin layer chromatography, Pd/C was filtered off. Then, after performing vacuum concentration, purification was performed using silica gel column chromatography (eluting solvent: ethyl acetate/toluene=1/2). The residue (128 g) was dissolved into 300 ml of dichloromethane and washed five times with 300 ml of ion exchanged water. Further, after vacuum concentration of the organic layer, by recrystallization using 310 ml of ethyl acetate, 25.87 g (47.0 mmol) of cholestanyl 2,4-diaminophenylpropionic acid (D2) was obtained (yield: 57%). Measurement result of ¹H-NMR of cholestanyl 2,4-diaminophenylpropionic acid is shown below.
¹H-NMR (400MHz, CDCl₃) δ: 6.80 ppm (1H, d, J = 7.8 Hz), 6.09 ppm (1H, d, J = 7.8), 6.04 ppm (1H, s), 4.69 ppm (1H, m), 3.595 ppm (4H, br s), 2.71 ppm (3H, t, J = 7.3 Hz), 2.54 ppm (3H, t, J = 7.3 Hz), 1.97 - 062 ppm (47H, m)

### Comparative Example 1 Synthesis of cholestanyl 3,5-diaminobenzoate (D3)

### (1) Synthesis of cholestanyl 3,5-dinitrobenzoate

After dissolving 49.17 g (136 mmol) of cholestanol in 150 ml of toluene, 25 ml of THF, and 17.01 g (168 mmol) of triethylamine (produced by Wako Pure Chemical Industries Co., Ltd.), the solution was ice cooled. Under ice cooling, the solution of 100 ml of toluene and 25 ml of THF, which contain 32.80 g (142 mmol) of 3,5-dinitorobenzoyl chloride (produced by Wako Pure Chemical industries, Ltd.), was dropped. The solution was stirred at room temperature for 4 hours, and after confirming disappearance of the raw material using thin layer chromatography, it was washed with 150 ml of ion exchanged water. After washing with 150 ml of sodium bicarbonate water, it was washed with 150 ml of ion exchanged water twice, and the organic layer was subjected to vacuum concentration. 187.5 ml of toluene and 562.5 ml of n-hexane were added to the residue (72.49 g), and it was warmed, dissolved and then cooled and it was subjected to crystallization. By stirring it at 5°C or lower for 1 hour and filtering out, 49.24 g of cholestanyl 3,5-dinitrobenzoate was obtained in a wet crystal state. And it was preceded to next step without drying. Measurement result of ¹H-NMR of cholestanyl 2,4-dinitrobenzoate is shown below.
¹H-NMR (400MHz, CDCl₃) δ: 9.21 ppm (1H, t, J = 2.0 Hz), 9.14 ppm (1H, d, J = 2.0 Hz), 5.07 ppm (1H, m), 2.00-0.69 ppm (47H, m)

### (2) Synthesis of cholestanyl 3,5-diaminobenzoate

Cholestanyl 3,5-dinitrobenzoate (24.62 g) obtained in the above (1) was dissolved into 125 ml of THF and 25 ml of methanol, and 1.25 g of 5% Pd/C (50% wet) was charged, and after replacement with, nitrogen, the container was replaced with hydrogen and the solution was stirred at 35 to 40°C for 1.5 hour and at 50°C for 2.5 hours. After confirming disappearance of the raw material using HPLC, Pd/C was filtered off, and the filtrate was passed through a short column of silica gel and activated alumina, and the solution was subjected to vacuum concentration. The residue (25.66 g) was dissolved into 50 ml of THF, 150 ml of IPA and 150 ml of acetonitrile under warming, and stirred at 60°C for 10 minutes. By cooling with water, stirring at 25°C for 30 minutes and under ice cooling for 1 hour, and then filtering out and drying, 17.08 g of cholestanyl 3,5-diaminobenzoate was obtained (yield: 51.7%). Measurement result of ¹H-NMR of cholestanyl 3,5-diaminophenyl acetate is shown below.
¹H-NMR (400MHz, CDCl₃) δ: 6.77 ppm (2H, d, J = 1.6 Hz), 6.16 ppm (1H, t, J = 1.6 Hz), 4.88 ppm (1H, m), 3.66 ppm (4H, br s), 1.99-0.69 ppm (47H, m)

### Comparative Example 2 Synthesis of cholestanyl 3,5-diaminophenylamide (D4)

50.38 g of cholestanyl 3 , 5-diaminophenylamide was obtained (yield: 71%) by a similar method as in Comparative Example 1, except by changing 49.17 g (136 mmol) of cholestanol to 52.72 g (136 mmol) of cholestanyl amine. Measurement result thereof of ¹H-NMR is shown below.
¹ H-NMR (400MHz, CDCl₃) δ: 6.43 ppm (2H, d, J = 2.0 Hz), 6.10 ppm (1H, t, J = 1.6 Hz), 5.81 ppm (1H, d, J = 8.0 Hz), 3.89 ppm (1H, m), 3.66 ppm (4H, br s), 1.99-0.69 ppm (47H, m)

### Example 3 Synthesis of polyimide polymer (PI-1)

After dissolving 2.157 g (4.0 mmol) of D1 obtained in Example 1 and 1.738 g (16.1 mmol) of p-phenylenediamine (produced by Wako Pure Chemical Industries Co., Ltd.) in 12.6 g of N-methylpyrrolidone (NMP), 4.504 g (20.1 mmol) of 2-carboxymethyl-1,3,4-cyclopentanetricarboxylic acid-1,4:2,3-dianhydride(tetracarboxylic acid dianhydride) was charged and by a reaction (polymerization) at 60°C for 2.5 hours, an NMP solution of a polyamic acid polymer was obtained. It should be noted that as for the tetracarboxylic acid dianhydride, the one synthesized in accordance with a method described in JP-A-58-109479 was used. To the NMP solution of the resultant polyamic acid polymer, 49 g of NMP was further added so that a concentration of the polyamic acid became 12% by weight, and then, 2.872 g (28.1 mmol) of acetic anhydride, and 3.179 g (40.2 mmol) of pyridine and 7.95g of NMP were added to prepare a 10 % by weight solution of the polyamic polymer. This solution was heated up to 110°C to carry out a dehydration ring closing reaction (imidation) of the polyamic acid polymer for 4 hours. After completion of the reaction, the reaction solution was concentrated at 80 to 90°C under a reduced pressure of 1 to 3 hPa, so as to attain half amount of the reaction solution, and then 80 ml of NMP was added. Further, the solution was concentrated at 80 to 90°C under a reduced pressure of 1 to 3 hPa, so as to attain half amount of the reaction solution. By this concentration, acetic anhydride and pyridine used in the reaction were removed, and an NMP solution of about 20% by weight of the polyimide polymer (PI-1) was obtained. The resultant PI-1 was diluted by adding NMP so as to attain 7% by weight, to measure viscosity of said solution. It should be noted that viscosity was measured using an E-type rotational viscometer (device name RE-80, manufactured by Tokisangyo Co., Ltd.) at a set temperature of 25°C. In addition, imidation ratio of the resultant polyimide was calculated from ratio of integrated value of benzene protons and integrated value of amide protons, using NMR (AL-400, manufactured by JEOL Ltd.). The obtained Viscosity and imidation ratio are shown in Table 1. It should be noted that viscosity and imidation ratio were measured and calculated similarly also in the following Examples and Comparative Examples.

### Example 4 Synthesis of polyimide polymer (PI-2)

After 1.202 g (2.2 mmol) of D1 obtained in Example 1, and 2.179 g (20.2 mmol) of p-phenylenediamine (produced by Wako Pure Chemical Industries Co. , Ltd.) were dissolved in 19.6 g of NMP, 5.019 g (22.4 mmol) of 2-carboxymethyl-1,3,4-cyclopentanetricarboxylic acid-1,4:2,3-dianhydride(tetracarboxylic acid dianhydride) (produced by Wako Pure Chemical Industries Co., Ltd.) was charged and by a reaction (polymerization) at 60°C for 1 hour, and an NMP solution of a polyamic acid polymer was obtained. Into the resultant NMP solution of the polyamic acid polymer, 42 g of NMP was added, so that concentration of the polyamic acid became 12% by weight, and then by adding 3.200 g (31.3 mmol) of acetic anhydride, 3.542 g (44.8 mmol) of pyridine and 7.26 g of NMP, a 10% by weight solution of the polyamic acid polymer was prepared. This solution was heated up to 110°C to carry out a dehydration ring closing reaction (imidation) of the polyamic acid polymer for 4 hours. After completion of the reaction, the reaction solution was concentrated at 80 to 90°C under a reduced pressure of 1 to 3 hPa, so as to attain half amount of the reaction solution, and then 80 ml of NMP was added. Further, the solution was concentrated at 80 to 90°C under a reduced pressure of 1 to 3 hPa, so as to attain half amount of the reaction solution. By this concentration, acetic anhydride and pyridine used in the reaction were removed, and an NMP solution of about 20% by weight of the polyimide polymer (PI-2) was obtained. Viscosity of the NMP solution of 7% by weight of said PI-2, along with imidation ratio of said polyimide polymer are shown in Table 1.

### Example 5 Synthesis of polyimide polymer (PI-3)

After 1.228 g (2.2 mmol) of D2 obtained in Example 2 and 2.171 g (20.1 mmol) of p-phenylenediamine (produced by Wako Pure Chemical Industries Co. , Ltd.) were dissolved in 13.7 g of NMP, 5.000 g (22.3 mmol) of 2-carboxymethyl-1,3,4-cyclopentanetricarboxylic acid-1,4:2,3-dianhydride(tetracarboxylic acid dianhydride) (produced by Wako Pure Chemical Industries Co., Ltd.) was charged and by a reaction (polymerization) for 3 hours, an NMP solution of a polyamic acid polymer was obtained. Into the resultant NMP solution of the polyamic acid polymer, 42 g of NMP was added, so that concentration of the polyamic acid became 12% by weight, and then by adding 3.188 g (31.2 mmol) of acetic anhydride, 3.529 g (44.6 mmol) of pyridine and 7.28 g of NMP, a 10% by weight solution of the polyamic acid polymer was prepared. This solution was heated up to 110°C to carry out a dehydration ring closing reaction (imidation) of the polyamic acid polymer for 4 hours. After completion of the reaction, the reaction solution was concentrated at 80 to 90°C under a reduced pressure of 1 to 3 hPa, so as to attain half amount of the reaction solution, and then 80 ml of NMP was added. Further, the solution was concentrated at 80 to 90°C under a reduced pressure of 1 to 3 hPa, so as to attain half amount of the reaction solution. By this concentration, acetic anhydride and pyridine used in the reaction were removed, and an NMP solution of about 20% by weight of the polyimide polymer (PI-3) was obtained. Viscosity of the NMP solution of 7% by weight of said PI-3, along with imidation ratio of said polyimide polymer are shown in Table 1.

### Comparative Example 3 Synthesis of polyimide polymer (PI-4)

After 2. 115 g (4.0 mmol) of the obtained D3 in Comparative Example 1, 1.750 g (16.2 mmol) of p-phenylenediamine (produced by Wako Pure Chemical Industries Co., Ltd.) were dissolved in 33.6 g of N-methylpyrrolidone (NMP), 4.5325 g (20.2 mmol) of 2-carboxymethyl-1,3,4-cyclopentanetricarboxylic acid-1,4:2,3-dianhydride(tetracarboxylic acid dianhydride) (produced by Wako Pure Chemical Industries Co., Ltd.) was charged and by a reaction (polymerization) at 60°C for 1. 5 hour, an NMP solution of a polyamic acid polymer was obtained. Into the resultant NMP solution of the polyamic acid polymer, 28 g of NMP was added, so that concentration of the polyamic acid became 12% by weight, and then by adding 2.891 g (28.3 mmol) of acetic anhydride, 3.200 g (40.5 mmol) of pyridine and 7.91 g of NMP, a 10% by weight solution of the polyamic acid polymer was prepared. This solution was heated up to 110°C to carry out a dehydration ring closing reaction (imidation) of the polyamic acid polymer for 4 hours. After completion of the reaction, the reaction solution was concentrated at 80 to 90°C under a reduced pressure of 1 to 3 hPa, so as to attain half amount of the reaction solution, and then 80 ml of NMP was added. Further, the solution was concentrated at 80 to 90°C under a reduced pressure of 1 to 3 hPa, so as to attain half amount of the reaction solution. By this concentration, acetic anhydride and pyridine used in the reaction were removed, and an NMP solution of about 20% by weight of the polyimide polymer (PI-4) was obtained. Viscosity of the NMP solution of 7% by weight of said PI-4, along with imidation ratio of said polyimide polymer are shown in Table 1.

### Comparative Example 4 Synthesis of olyimide polymer (PI-5)

After 2.112 g (4.0 mmol) of the obtained D4 in Comparative Example 2, 1.751 g (16.2 mmol) of p-phenylenediamine (produced by Wako Pure Chemical Industries Co., Ltd.) were dissolved in 33.6 g of NMP, 4.537 g (20.2 mmol) of 2-carboxymethyl-1,3,4-cyclopentanetricarboxylic acid-1,4:2,3-dianhydride(tetracarboxylic acid dianhydride) (produced by Wako Pure Chemical Industries Co., Ltd.) was charged and by a reaction (polymerization) at 60°C for 1 hour, an NMP solution of a polyamic acid polymer was obtained. Into the resultant NMP solution of the polyamic acid polymer, 28 g of NMP was added, so that concentration of the polyamic acid became 12% by weight, and then by adding 2.893 g (28.3 mmol) of acetic anhydride, 3.202 g (40.5 mmol) of pyridine and 7.91 g of NMP, a 10% by weight solution of the polyamic acid polymer was prepared. This solution was heated up to 110°C to carry out a dehydration ring closing reaction (imidation) of the polyamic acid polymer for 4 hours. After completion of the reaction, the reaction solution was concentrated at 80 to 90°C under a reduced pressure of 1 to 3 hPa, so as to attain half amount of the reaction solution, and then 80 ml of NMP was added. Further, the solution was concentrated at 80 to 90°C under a reduced pressure of 1 to 3 hPa, so as to attain half amount of the reaction solution. By this concentration, acetic anhydride and pyridine used in the reaction were removed, and an NMP solution of about 20% by weight of the polyimide polymer (PI-5) was obtained. Viscosity of the NMP solution of 7% by weight of said PI-5, along with imidation ratio of said polyimide polymer are shown in Table 1.

**Table 1**

| | Polymer | Tetracarboxylic acid dianhydride | | Diamine compound | | Imidation ratio (%) | Viscosity (mPa·s) |
|---|---|---|---|---|---|---|---|
| | | Abbreviation | Molar fraction | Abbreviation | Molar fraction | | |
| Example 3 | PI-1 | TC-1* | 0.5 | D1 | 0.1 | 51 | 13 |
| | | | | PDA* | 0.4 | | |
| Example 4 | PI-2 | TC-1* | 0.5 | D1 | 0.05 | 50 | 16 |
| | | | | PDA* | 0.45 | | |
| Example 5 | PI-3 | TC-1* | 0.5 | D2 | 0.05 | 47 | 9.2 |
| | | | | PDA* | 0.45 | | |
| Comparative Example 3 | PI-4 | TC-1* | 0.5 | D3 | 0.1 | 60 | 20 |
| | | | | PDA* | 0.4 | | |
| Comparative Example 4 | PI-5 | TC-1* | 0.5 | D4 | 0.1 | 57 | 22 |
| | | | | PDA* | 0.4 | | |

| | | | | | | | |
|---|---|---|---|---|---|---|---|
| TC-1: 2-carboxymethyl-1,3,4-cyclopentanetricarboxylic acid-1,4:2,3-dianhydride PDA: p-phenylenediamine | | | | | | | |

### Example 6 Preparation of liquid crystal alignment film using the PI-1, along with pre-tilt angle and voltage retention rate thereof

To 3.44 g of the resultant polyimide solution (PI-1), obtained in Example 3, 0.06 g of N,N,N',N'-tetraglycidyl-4,4'-diaminodiphenylmethane (MY-721) (equivalent to 10 parts by weight relative to 100 parts by weight of PI-1) was added, as a cross-linking agent, and 5.60 g of NMP and 8.4 g of BC were further added so as to attain mixing ratio of NMP/BC=50/50 (weight ratio) [NMP: N-methyl-2-pyrrolidone, BC: butylcellosolve (ethylene glycol monobutyl ether)] to prepare a solution having a solid concentration of 4% by weight. Then, the solution was filtered using a filter having a pore size of 0.5 µm, to obtain the liquid crystal aligning agent relevant to the present invention.

A liquid crystal display element was prepared using the resultant liquid crystal aligning agent, and UV resistance of a liquid crystal alignment film was evaluated by measuring voltage retention rate in each of the case where it was prepared without UV irradiation and the case where it was prepared under UV irradiation on the alignment film surface. Specifically, the obtained liquid crystal aligning agent was coated on a transparent conductive film composed of an ITO film (indium tin oxide film) installed at one surface of a glass substrate having a thickness of 1 mm, by a spinner, at 70°C for 2 minutes, and which was further dried at 220°C for 20 minutes, to form a coated film having a dried film thickness of 70 nm. Then, rubbing processing was performed using a rubbing machine (manufactured by E. H. C. Co., Ltd.) having a roll wound with rayon cloth, to furnish liquid crystal aligning capability to the relevant coated film, and obtain the liquid crystal alignment film. Condition of said rubbing processing was as follows: a roller rotation number of 300 rpm, a stage moving velocity of 600 mm/min, and pile push-in length of 0.1 mm. In the case where the display element was prepared under UV irradiation, UV light having a UV intensity of 300 mW/cm² was irradiated for 133.3 second (40 J) onto the alignment film surface formed.

Two pieces of substrates formed with the liquid crystal alignment film by the above-described way were prepared, and after coating epoxy resin-type adhesives containing a 10 µm of gap agent at the outer peripheral part of each substrate, the two substrates were arranged in an opposing way via the gap to abut and crimp the outer peripheral parts themselves to harden the adhesives.

Then, after filling a negative-type liquid crystal (ZLI-4792, produced by Merck kGaA) from a liquid crystal injecting port, installed in advance, into the inside of the cell gap between the substrates for the liquid crystal display element by utilization of capillary phenomena, by sealing the injecting entrance and exit with photo-curing adhesives, the liquid crystal display element was prepared. Using the resultant liquid crystal display element, pre-tilt angle and voltage retention rate were measured. Results thereof are shown in Table 2.

### Example 7 Synthesis of liquid crystal alignment film using the PI-2, along with pre-tilt angle and voltage retention rate thereof

Pre-tilt angle and voltage retention rate of a liquid crystal display element were obtained in the case of using the PI-2, by performing an experiment similarly as in Example 6, except by using 3.01 g of the PI-2 instead of 3.44 g of the PI-1. Results thereof together with results of Example 6 are shown in Table 2.

### Example 8 Synthesis of liquid crystal alignment film using the PI-3, along with pre-tilt angle and voltage retention rate thereof

Pre-tilt angle and voltage retention rate of a liquid crystal display element were obtained in the case of using the PI-3, by performing an experiment similarly as in Example 6, except by using 3.93 g of the PI-3 instead of 3.44 g of the PI-1, and preparing a liquid crystal aligning agent so as to attain a solid content concentration of 7%. Results thereof together with results of Example 6 are shown in Table 2.

### Comparative Example 5 Synthesis of liquid crystal alignment film using the PI-4, along with pre-tilt angle and voltage retention rate thereof

Pre-tilt angle and voltage retention rate of a liquid crystal display element were obtained in the case of using the PI-4, by performing an experiment similarly as in Example 6, except by using 3.83 g of the PI-4 instead of 3.44 g of the PI-1. Results thereof together with results of Example 6 are shown in Table 2.

### Comparative Example 6 Synthesis of liquid crystal alignment film using the PI-5, along with pre-tilt angle and voltage retention rate thereof

Pre-tilt angle and voltage retention rate of a liquid crystal display element were obtained in the case of using the PI-5, by performing an experiment similarly as in Example 6, except by using 3.82 g of the PI-5 instead of 3.44 g of the PI-1. Results thereof together with results of Example 6 are shown in Table 2.

**Table 2**

| Examples | Polymer | No UV irradiation | | Irradiation of 40J UV | |
|---|---|---|---|---|---|
| | (Polymer concentration) | Voltage retention rate (%) | Pre-tilt angle (°) | Voltage retention rate (%) | Pre-tilt angle (°) |
| Example 6 | PI-1 (20mol%) | 93 | 89 | 87 | 89 |
| Example 7 | PI-2 (10mol%) | 73 | 89 | 66 | 89 |
| Example 8 | PI-3 (10mol%) | 86 | 89 | 78 | 89 |
| Comparative Example 5 | PI-4 (20mol%) | 70 | 89 | 38 | 89 |
| Comparative Example 6 | PI-5 (20mol%) | 61 | 89 | 35 | 89 |

According to the above results, the liquid crystal alignment films obtained by using the polyimide (PI-1 and PI-2) obtained by using the cholestanyl 2,4-diaminophenylacetate (D1), and the polyimide (PI-3) obtained by using the cholestanyl 2,4-diaminophenylpropionate (D2) showed a voltage retention rate of at least 70% or higher, in the case of no UV irradiation, and a voltage retention rate of 60% or higher even under UV irradiation. On the other hand, the liquid crystal alignment films obtained by using the polyimide (PI-4 to 5) obtained by using the cholestanyl 3,5-diaminobenzoate (D3) or cholestanyl 3,5-diaminophenylamide (D4), which is conventional products, showed a voltage retention rate of 60 to 70% in the case of no UV irradiation, however, both showed a low voltage retention rate of 40% or lower in the case of UV irradiation. That is, it has been confirmed that the liquid crystal alignment films obtained by using the polyimide obtained by using the compound represented by the general formula [1] of the present invention show higher voltage retention rate as compared with conventional products, and show less deterioration even after strong UV irradiation.

Further, it has also been confirmed that the polyimide obtained by using the compound represented by the general formula [1] of the present invention shows pre-tilt angle equivalent to that of conventional products, because pre-tilt angle of the liquid crystal alignment films of Examples and Comparative Examples was 89°.

## Claims

1. A compound represented by the following general formula [1] : 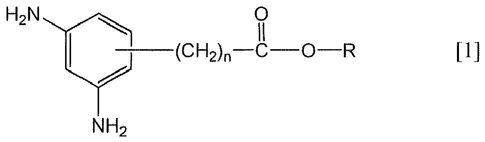 wherein n represents an integer of 1 to 6, and R represents an alkyl group having 8 to 20 carbon atoms or a group having a steroid skeleton.

2. The compound according to claim 1, wherein R is a group having a steroid skeleton.

3. The compound according to claim 1, wherein n is an integer of 1 to 3.

4. A polyamic acid or a polyimide obtained by a reaction of the following compounds (A), (B) and (C):
(A) compound represented by the following general formula [1] ; 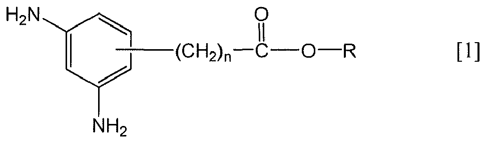 wherein n represents an integer of 1 to 6, and R represents an alkyl group having 8 to 20 carbon atoms or a group having a steroid skeleton,
(B) compound represented by the following general formula [2] ; 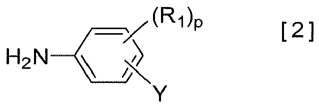 wherein R₁ represents an alkyl group having 1 to 6 carbon atoms, or an arylalkyl group having 7 to 12 carbon atoms; p represents an integer of 0 to 4; and Y represents an amino group or a 4-aminophenyl group,
(C) compound represented by the following general formula [3] or [4] ; 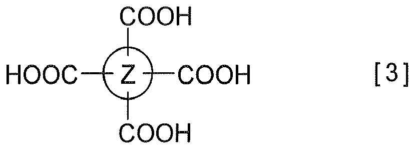 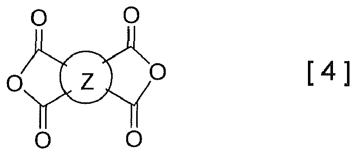 wherein Z represents a tetravalent hydrocarbon group.

5. The polyamic acid or a polyimide according to claim 4 , wherein R is a group having a steroid skeleton.

6. The polyamic acid or a polyimide according to claim 4, wherein n is an integer of 1 to 3.

7. A liquid crystal aligning agent comprising the polyamic acid or the polyimide obtained by a reaction of the following compounds (A), (B) and (C):
(A) compound represented by the following general formula [1] ; 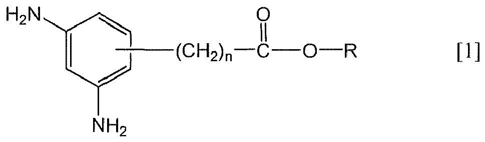 wherein n represents an integer of 1 to 6, and R represents an alkyl group having 8 to 20 carbon atoms or a group having a steroid skeleton,
(B) compound represented by the following general formula [2] ; 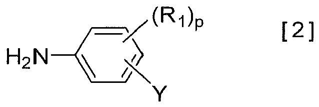 wherein R₁ represents an alkyl group having 1 to 6 carbon atoms, or an arylalkyl group having 7 to 12 carbon atoms, p represents an integer of 0 to 4, and Y represents an amino group, a 4-aminophenyl group or a 4-aminobenzyl group),
(C) compound represented by the following general formula [3] or [4] : 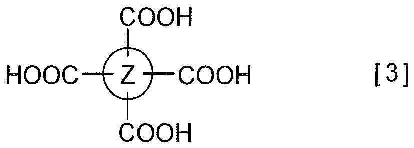 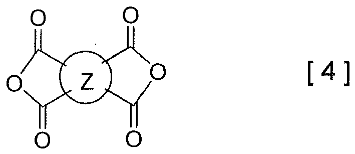 wherein Z represents a tetravalent hydrocarbon group.

8. The liquid crystal aligning agent according to claim 7, wherein R is a group having a steroid skeleton.

9. The liquid crystal aligning agent according to claim 7, wherein n is an integer of 1 to 3.
